# EUROPÄISCHE PATENTSCHRIFT

(11) **EP 2 890 770 B1**
(45) Veröffentlichungstag und Bekanntmachung des Hinweises auf die Patenterteilung: **31.01.2018**
(21) Anmeldenummer: 13753866.6
(22) Anmeldetag: 30.08.2013
(51) Int. Cl.: C11D 1/02, C11D 3/37, C11D 3/50, C11D 17/00, C11D 1/10, C11D 1/14, C11D 1/28, C11D 1/29, C11D 1/37

(54) **WC-GEL**
TOILET GEL
GEL WC

(30) Priorität: 31.08.2012 DE 102012215536
(43) Veröffentlichungstag der Anmeldung: 08.07.2015
(73) Patentinhaber: Henkel AG & Co. KGaA, 40589 Düsseldorf (DE)
(72) Erfinder: SCHIEDEL, Marc-Steffen, 40789 Monheim (DE); PLANTIKOW, Petra, 40627 Düsseldorf (DE); HOCHKUGLER, Sabine, A-1230 Wien (AT); REICHERT, Christian, 40476 Düsseldorf (DE)
(86) Internationale Anmeldenummer: PCT/EP2013/067989
(87) Internationale Veröffentlichungsnummer: WO 2014/033259

(56) Entgegenhaltungen:
- EP-A1- 2 275 524
- WO-A1-02/26925
- WO-A1-2012/113671
- DE-A1-102010 028 352
- US-A1- 2012 178 824

## Beschreibung

Die Erfindung betrifft eine haftende gelförmige oder pastöse Zusammensetzung zur Reinigung und/oder Beduftung eines WCs. Diese Zusammensetzung wird innen auf die WC-Keramik aufgetragen und erst nach mehreren Spülvorgängen abgespült.

WC-Duftspüler werden bereits seit langem zur Reinigung, Desinfektion und Beduftung von Toiletten verwendet. In ihrer ursprünglichen Form werden sie als feste Blocks unter dem Beckenrand (sogenannte Rimblocks) sowie im Wasserkasten (In-tank-Blocks oder cistern blocks) eingesetzt. Dabei haben in den letzten Jahren die Ästhetik und die Leistung eine immer größere Bedeutung erlangt. Dies führte beispielsweise zur Entwicklung gelförmiger oder flüssiger Duftspüler, die teilweise in Mehrkammerbehältnissen angeboten werden und so die Kombination eines Reinigungsmittels, welches bei Betätigung der WC-Spülung abgegeben wird, mit einer permanenten Raumbeduftung erlauben.

Herkömmliche feste, flüssige oder auch gelförmige Duftspüler werden dabei mittels entsprechender Vorrichtungen, der sogenannten WC-Körbchen, in die Spültoilette eingebracht. Diese WC-Körbchen werden von manchen Verbrauchern jedoch aus hygienischen Gründen abgelehnt. Zum einen kann im Verlauf der Lebensdauer des Produkts eine Besiedelung mit Keimen erfolgen, was zur Ausbildung eines unschönen Biofilms führen kann. Zum anderen wird ein Nachfüllen oder Austauschen des Körbchens aufgrund der notwendigen Berührung als unhygienisch oder gar ekelerregend empfunden, selbst wenn kein sichtbarer Biofilm an der Vorrichtung haftet. Und schließlich empfinden einige Verbraucher es auch als nachteilig, dass das Körbchen bei der WC-Reinigung mittels WC-Bürste verschoben werden kann.

Es wurden daher selbsthaftende Mittel entwickelt, die direkt auf die WC-Keramik appliziert werden und nach und nach abgespült werden. EP 1086199 B1 beschreibt beispielsweise ein festes oder pastöses, haftendes Sanitärmittel, welches Wasser, anionische und/oder nichtionische und/oder amphotere Tenside, Duftstoffe, einen Haftvermittler sowie gegebenenfallsweitere übliche Bestandteile umfasst, wobei der Haftvermittler ausgewählt sein soll aus der Gruppe bestehend aus Polyalkoxyalkanen, Cellulosen, Stärke, Alginaten, Diurethanen, Gelatine, Pectinen, Oleylaminen, Alkyldimethylaminoxiden, Stearaten, Natriumdodecylbenzolsulfonat, Agar-Agar, Gummi arabicum, Johannisbrotkernmehl, Polyacrylat, Polyvinylalkohol und Polyvinylpyrrolidon. Ähnliche Pasten werden auch in EP1318191 B1 beschrieben, in der jedoch der Haftvermittler aus der Gruppe der Oligo- oder Polyethylenoxid und/oder Oligo-und/oder Polypropylenoxid und/oder Oligo- und/oder Polybutylenoxid umfassenden Blockcopolymere ausgewählt sein soll. EP 1325103 B1 beschreibt ebenfalls solche Pasten mit einem Haftvermittler aus der aus Polyalkoxyalkanen, Alginaten, Diurethanen, Gelatine, Pectinen, Oleylaminen, Alkyldimethylaminoxiden, Stearaten, Sulfonaten, Sulfaten und Carbonaten bestehenden Gruppe, die zusätzlich entweder wenigstens eine aus der Gruppe Glycerin, 1,3-Dihydroxypropan, 1,3- oder 1,4-Dihydroxybutan, 1,3-Dihydroxyisobutan, Pentaerythrit ausgewählte Verbindung oder zwischen 1 und 20 Gew.-% einer aliphatischen Di-, Oligo- oder Polyhydroxyverbindung oder deren Ether umfasst.

Es wurde nun gefunden, dass eine haftende gelförmige oder pastöse Zusammensetzung formuliert werden kann, die mindestens ein Tensid aus der Gruppe bestehend aus Fettalkoholsulfaten, Fettalkoholethersulfaten, Alkylsulfonaten, Sulfo-Estoliden, Methylestersulfonat, Acylaminoalkansulfonaten (Fettsäuretauriden), Sulfosuccinaten und Gemischen derselben sowie Parfüm und Wasser enthält, und die weiterhin 0,1 bis 80 Gew.-% eines Esters der Polyisobutenbernsteinsäure enthält und/oder frei von aliphatischen Verbindungen mit mehr als einer OH-Gruppe sowie von weiteren Tensiden ist. Dieses Produkt weist eine transparente und klare Ästhetik auf und besitzt nach der Applikation eine gute Formstabilität über den gesamten Abspülzyklus. Auch wenn die Zusammensetzung durch fehlende Betätigung der WC-Spülung über einen Zeitraum von einigen Stunden nicht von Wasser umspült wird, verändert sie nicht die Form oder wird unansehnlich.

Gegenstand der Erfindung ist daher eine haftende gelförmige oder pastöse Zusammensetzung zur Reinigung und/oder Beduftung eines WCs, welche innen auf die WC-Keramik aufgetragen und erst nach mehreren Spülvorgängen abgespült wird, enthaltend mindestens ein Tensid aus der Gruppe bestehend aus Fettalkoholsulfaten, Fettalkoholethersulfaten, Alkylsulfonaten, Sulfo-Estoliden, Methylestersulfonaten, Acylaminoalkansulfonaten (Fettsäuretauriden), Sulfosuccinaten und Gemischen derselben, sowie Parfüm und Wasser, die weiterhin 0,1 bis 80 Gew.-% eines Esters der Polyisobutenbernsteinsäure enthält und/oder frei von aliphatischen Verbindungen mit mehr als einer OH-Gruppe sowie von weiteren Tensiden ist.

Diese gelförmige oder pastöse Zusammensetzung wird vorteilhafterweise mit einem Applikator aufgetragen. In einer Ausführungsform werden zwei gelförmige oder pastöse Formulierungen in einen Zweikammerapplikator eingebracht, wie er auch in der Patentanmeldung DE 102010028352.5 beschrieben wird.

Die Begriffe "gelförmig" und pastös" haben dabei erfindungsgemäß die dem Fachmann bekannten Bedeutungen.

Vorzugsweise wird erfindungsgemäß unter einer "gelförmigen" Zusammensetzung eine Zusammensetzung verstanden, die eine Fließgrenze aufweist, die unter Anwendungsbedingungen bei 25°C durch die Erdanziehungskraft nicht überwunden wird, und deren Speichermodul bei einer Schubdeformation von 1%, gemessen mittels eines Rotationsrheometers (oszillierende Messung, 1 Hz, Platte-Platte, cross-hatched), bei 25°C einen höheren Wert aufweist als der zugehörige Verlustmodul.

Vorzugsweise wird erfindungsgemäß unter einer "pastösen" Zusammensetzung eine Zusammensetzung verstanden, die eine Fließgrenze aufweist, die unter Anwendungsbedingungen bei 25°C durch die Erdanziehungskraft nicht überwunden wird, und deren Speichermodul bei einer Schubdeformation von 0,5‰, gemessen mittels eines Rotationsrheometers (oszillierende Messung, 1 Hz, Platte-Platte, cross-hatched), bei 25°C einen höheren Wert aufweist als der zugehörige Verlustmodul.

Stoffe, die auch als Inhaltsstoffe von kosmetischen Mitteln dienen, werden nachfolgend gegebenenfalls gemäß der *International Nomenclature Cosmetic Ingredient* (INCI)-Nomenklatur bezeichnet. Chemische Verbindungen tragen eine INCI-Bezeichnung in englischer Sprache, pflanzliche Inhaltsstoffe werden ausschließlich nach Linne in lateinischer Sprache aufgeführt, sogenannte Trivialnamen wie "Wasser", "Honig" oder "Meersalz" werden ebenfalls in lateinischer Sprache angegeben. Die INCI-Bezeichnungen sind dem International Cosmetic Ingredient Dictionary and Handbook- Seventh Edition (1997) zu entnehmen, das von The Cosmetic, Toiletry, and Fragrance Association (CTFA), 1101 17th Street, NW, Suite 300, Washington, DC 20036, USA, herausgegeben wird und mehr als 9.000 INCI-Bezeichnungen sowie Verweise auf mehr als 37.000 Handelsnamen und technische Bezeichnungen einschließlich der zugehörigen Distributoren aus über 31 Ländern enthält. Das *International Cosmetic Ingredient Dictionary and Handbook* ordnet den Inhaltsstoffen eine oder mehrere chemische Klassen (*Chemical Classes*), beispielsweise *Polymeric Ethers,* und eine oder mehrere Funktionen (*Functions*), beispielsweise *Surfactants - Cleansing Agents,* zu, die es wiederum näher erläutert und auf die nachfolgend gegebenenfalls ebenfalls Bezug genommen wird.

Die Angabe *CAS* bedeutet, dass es sich bei der nachfolgenden Zahlenfolge um eine Bezeichnung des *Chemical Abstracts Service* handelt.

Im Rahmen der vorliegenden Erfindung stehen Fettsäuren beziehungsweise Fettalkohole beziehungsweise deren Derivate - soweit nicht anders angegeben - stellvertretend für verzweigte oder unverzweigte Carbonsäuren beziehungsweise Alkohole beziehungsweise deren Derivate mit vorzugsweise 6 bis 22 Kohlenstoffatomen, insbesondere 8 bis 20 Kohlenstoffatomen, besonders bevorzugt 10 bis 18 Kohlenstoffatomen, äußerst bevorzugt 12 bis 16 Kohlenstoffatomen, beispielsweise 12 bis 14 Kohlenstoffatomen. Erstere sind insbesondere wegen ihrer pflanzlicher Basis als auf nachwachsenden Rohstoffen basierend aus ökologischen Gründen bevorzugt, ohne jedoch die erfindungsgemäße Lehre auf sie zu beschränken. Insbesondere sind auch die beispielsweise nach der ROELENschen Oxo-Synthese erhältlichen Oxo-Alkohole beziehungsweise deren Derivate mit vorzugsweise 7 bis 19 Kohlenstoffatomen, insbesondere 9 bis 19 Kohlenstoffatomen, besonders bevorzugt 9 bis 17 Kohlenstoffatomen, äußerst bevorzugt 11 bis 15 Kohlenstoffatomen, beispielsweise 9 bis 11, 12 bis 15 oder 13 bis 15 Kohlenstoffatomen, entsprechend einsetzbar.

### Tenside

Das erfindungsgemäße Mittel enthält mindestens ein Tensid aus der Gruppe bestehend aus Fettalkoholsulfaten, Fettalkoholethersulfaten, Alkylsulfonaten, Sulfo-Estoliden, Methylestersulfonaten, Acylaminoalkansulfonaten (Fettsäuretauriden), Sulfosuccinaten und Gemischen derselben.

Bevorzugt im Rahmen der vorliegenden Erfindung sind die Fettalkoholsulfate und/oder Fettalkoholethersulfate, insbesondere die Fettalkoholsulfate. Fettalkoholsulfate sind Produkte von Sulfatierreaktionen an entsprechenden Alkoholen, während Fettalkoholethersulfate Produkte von Sulfatierreaktionen an alkoxylierten Alkoholen sind. Dabei versteht der Fachmann allgemein unter alkoxylierten Alkoholen die Reaktionsprodukte von Alkylenoxid, bevorzugt Ethylenoxid, mit Alkoholen, im Sinne der vorliegenden Erfindung bevorzugt mit längerkettigen Alkoholen. In der Regel entsteht aus n Molen Ethylenoxid und einem Mol Alkohol, abhängig von den Reaktionsbedingungen, ein komplexes Gemisch von Additionsprodukten unterschiedlicher Ethoxylierungsgrade. Eine weitere Ausführungsform der Alkoxylierung besteht im Einsatz von Gemischen der Alkylenoxide, bevorzugt des Gemisches von Ethylenoxid und Propylenoxid. Bevorzugte Fettalkoholethersulfate sind die Sulfate niederethoxylierter Fettalkohole mit 1 bis 4 Ethylenoxideinheiten (EO), insbesondere 1 bis 2 EO, beispielsweise 1,3 EO.

Alkylsulfonate sind weitere bevorzugte Tenside im Rahmen der Erfindung. Die Alkylsulfonate (INCI Sulfonic Acids) weisen üblicherweise einen aliphatischen geradkettigen oder ein- oder mehrfach verzweigten, acyclischen oder cyclischen, gesättigten oder ein- oder mehrfach ungesättigten Alkylrest mit 6 bis 22, vorzugsweise 9 bis 20, insbesondere 11 bis 18 und besonders bevorzugt 14 bis 17 Kohlenstoffatomen auf. Bevorzugte Alkylsulfonate weisen verzweigte, acyclische, gesättigte Alkylreste mit 6 bis 22, vorzugsweise 9 bis 20, insbesondere 11 bis 18 und besonders bevorzugt 14 bis 17 Kohlenstoffatomen auf.

Bevorzugte Alkylsulfonate sind die gesättigten Alkansulfonate, die ungesättigten Olefinsulfonate und die - sich formal von den auch den Alkylethersulfaten zugrundeliegenden alkoxylierten Alkoholen ableitenden - Ethersulfonate, bei denen man endständige Ethersulfonate

(n-Ethersulfonate) mit an die Polyether-Kette gebundener Sulfonat-Funktion und innenständige Ethersulfonate (i-Ethersulfonate) mit mit dem Alkylrest verknüpfter Sulfonat-Funktion unterscheidet.

Erfindungsgemäß besonders bevorzugt sind die Alkansulfonate, insbesondere die verzweigten Alkansulfonate, ganz besonders die sek. Alkansulfonate. Ein bevorzugtes Beispiel für die sek. Alkansulfonate ist das sekundäre Alkansulfonat sek. Na-C₁₃₋₁₇-Alkansulfonat (INCI Sodium C14-17 Alkyl Sec Sulfonate).

Weiterhin geeignet sind Sulfo-Estolide, wie sie beispielsweise in EP 2277860 A1 beschrieben sind, sowie Methylestersulfonate, Acylaminoalkansulfonate (Fettsäuretauride), Fettsäuresarcosinate, Sulfosuccinate und Gemische.

Die anionischen Tenside werden vorzugsweise als Natriumsalze eingesetzt, können aber auch als andere Alkali- oder Erdalkalimetallsalze, beispielsweise Magnesiumsalze, sowie in Form von Ammonium- oder Mono-, Di-, Tri- beziehungsweise Tetraalkylammoniumsalzen enthalten sein, im Falle der Sulfonate auch in Form ihrer korrespondierenden Säure.

Das erfindungsgemäße Mittel enthält das mindestens eine Tensid in einer Menge von 10 bis 80 Gew.-%, vorzugsweise 20 bis 60 Gew.-%, besonders bevorzugt 25 bis 55 Gew.-%, jeweils bezogen auf die gesamte haftende Zusammensetzung, wobei sich diese Angaben auf die Gesamtmenge an erfindungsgemäßem Tensidsystem beziehen.

### Polyisobutenbernsteinsäureester

In der erfindungsgemäßen Zusammensetzung wird weiterhin vorzugsweise ein Ester der Polyisobutenbernsteinsäure eingesetzt, wie er in der Patentanmeldung EP 11150613.5 beschrieben wird. Unter Polyisobutenbernsteinsäure versteht man oligomere oder polymere Makromoleküle mit einem Oligomerrest beziehungsweise Polymerrest, der von Isobuten abgeleitet ist und der an einem seiner Termini 1 oder 2 von Bernsteinsäure abgeleitete Reste, also Reste der Formel BS

-CH(COOH)CH₂COOH (BS)

und dementsprechend 2 oder 4 Carboxylgruppen aufweist, sowie Gemische davon.

Polyisobutenbernsteinsäuren können daher durch die folgende Formeln IIa und IIb beschrieben werden:

PIB-CH(COOH)CH₂COOH (IIa)

PIB'-[CH(COOH)CH₂COOH]₂ (IIb)

wobei PIB in Formel IIa für einen einwertigen, von Polyisobuten abgeleiteten Oligomerrest beziehungsweise Polymerrest und PIB' in Formel IIb für einen zweiwertigen, von Polyisobuten abgeleiteten Oligomerrest beziehungsweise Polymerrest stehen.

In den erfindungsgemäß verwendeten Estern der Polyisobutenbernsteinsäure liegt wenigstens eine der Carboxylgruppen in Form des Esters mit einem Poly-C₂-C₄-alkylenglykol oder einem Pols-C₂-C₄-alkylenglykolmono-C₁-C₂ᵣalkylether vor. Derartige Ester lassen sich durch die allgemeinen Formeln la und Ib beschreiben: 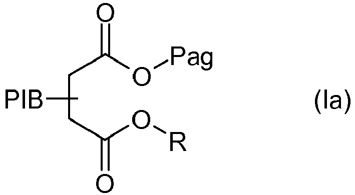 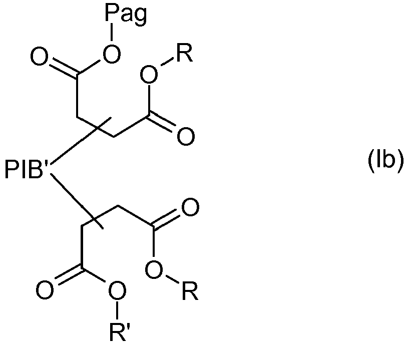 worin PIB und PIB' die zuvor für Formeln IIa und IIb angegebenen Bedeutungen aufweisen, R und R' unabhängig voneinander für Wasserstoff oder Pag stehen und Pag für einen von einem Pols-C₂-C₄-alkylenglykol oder einem Poly-C₂-C₄-alkylenglykolmono-C₁-C₂₂-alkylether abgeleiteten Rest stehen. In den Formeln la und Ib steht R insbesondere für Wasserstoff.

Unter Poly-C₂-C₄-alkylenglykolen versteht man lineare oder verzweigte Oligomere oder Polymere, die im Wesentlichen aus Wiederholungseinheiten der Formel -A-O- (im Folgenden auch Alkylenoxid-Wiederholungseinheiten) aufgebaut sind, worin A für C₂-C₄-Alkandiyl steht, und die an ihren Termini Hydroxylgruppen aufweisen.

Unter Poly-C₂-C₄-alkylenglykolmono-C₁-C₂₂-alkylethern versteht man lineare oder verzweigte Oligomere oder Polymere, die im Wesentlichen aus Wiederholungseinheiten der Formel -A-O-aufgebaut sind, worin A für C₂-C₄-Alkandiyl steht, die an einem ihrer Enden eine über Sauerstoff gebundene C₁-C₂₂-Alkylgruppe aufweisen und die an dem anderen Terminus beziehungsweise den anderen Termini Hydroxylgruppen aufweisen.

In diesen Poly-C₂-C₄-alkylenglykolen beziehungsweise Poly-C₂-C₄-alkylenglykolmono-C₁-C₂₂-alkylethern können die Wiederholungseinheiten der Formel -A-O- gleich oder verschieden sein. Sofern die Poly-C₂-C₄-alkylenglykole beziehungsweise Poly-C₂-C₄-alkylenglykolmono-C₁-C₂₂-alkylether verschiedene Wiederholungseinheiten der Formel -A-O- aufweisen, können diese statistisch, alternierend oder in mehreren, zum Beispiel 2, 3 oder 4 Blöcken angeordnet sein. In einer bestimmten Ausführungsform der Erfindung weisen die von den Poly-C₂-C₄-alkylenglykolen beziehungsweise Poly-C₂-C₄-alkylenglykolmono-C₁-C₂₂-alkylethern unterschiedliche Wiederholungseinheiten der Formel -A-O- auf, die statistisch angeordnet sind.

C₂-C₄-Alkandiyl steht in diesem Zusammenhang für einen gesättigten divalenten Kohlenwasserstoffrest mit 2 bis 4 C-Atomen wie 1,2-Ethandiyl, 1,2-Propandiyl, 1,3-Propandiyl, 1,4-Butandiyl, 1,2-Butandiyl, 1,3-Butandiyl, 2,3-Butandiyl oder 1-Methyl-1,2-propandiyl.

C₁-C₂₂-Alkyl steht in diesem Zusammenhang für einen gesättigten, acyclischen monovalenten Kohlenwasserstoffrest mit 1 bis 22 C-Atomen, insbesondere mit 1 bis 8 C-Atomen oder 1 bis 4 C-Atomen wie Methyl, Ethyl, n-Propyl, Isopropyl, 1-Butyl, 2-Butyl, tert.-Butyl, Isobutyl, n-Pentyl, Isopentyl, n-Hexyl, Isohexyl, n-Heptyl, Isoheptyl, n-Octyl, Isooctyl, 2-Ethylhexyl, n-Nonyl, Isononyl, n-Decyl, 2-Propylheptyl, n-Undecyl, n-Dodecyl, n-Tridecyl, Myristyl, Pentadecyl, Palmityl (= Cetyl), Heptadecyl, Octadecyl, Nonadecyl, Arachinyl oder Behenyl.

Unter von Isobuten abgeleiteten Polymerresten, im Folgenden auch Polyisobutenyl-Reste, versteht man organische Reste, die von linearen oder verzweigten Oligomeren beziehungsweise Polymeren des Isobutens abgeleitet sind und die bis zu 20 Gew.-%, vorzugsweise nicht mehr als 10 Gew.-% von Isobuten verschiedene C₂-C₁₂-Olefine, wie 1-Buten, 2-Buten, 2-Methyl-1-buten, 2-Methylpenten-1, 2-Methylhexen-1, 2-Ethylpenten-1, 2-Ethylhexen-1, 2-propylhepten-1, einpolymerisiert enthalten können. Derartige Reste können im Falle einwertiger Reste PIB beispielsweise durch die folgenden Formeln 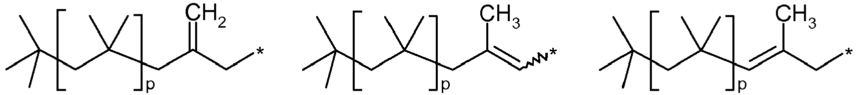 beziehungsweise im Falle zweiwertiger Reste PIB' beispielsweise durch die folgenden Formeln beschrieben werden 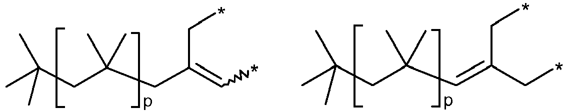 worin der Wert p+2 dem Polymerisationsgrad enspricht und die Anzahl an Isobuten-Einheiten im Polyisobuten-Rest angibt und * die Anknüpfung an den Bernsteinsäure(ester)-Rest bedeutet. In diesen Formeln kann ein Teil der Isobuteneinheiten -CH₂C(CH₃)₂-, in der Regel nicht mehr als 20 Gew.-%, vorzugsweise nicht mehr als 10 Gew.-%, durch davon verschiedene, von C₂-C₁₂-Olefinen abgeleitete C₂-C₁₂-Alkan-1,2-diylgruppen ersetzt sein. Der Polymerisationsgrad p+2 liegt typischerweise im Bereich von 5 bis 100, insbesondere im Bereich von 8 bis 80 und speziell im Bereich von 15 bis 65.

Im Hinblick auf die erfindungsgemäßen haftenden Zusammensetzungen sind solche Ester der Polyisobutenbernsteinsäure bevorzugt, die, bezogen auf das Gesamtgewicht des Esters, zu wenigstens 50 Gew.-%, insbesondere zu wenigstens 70 Gew.-% aus Estern der Formel la bestehen. Vorzugsweise enthalten die Ester der Polyisobutenbernsteinsäure, bezogen auf das Gesamtgewicht des Esters, weniger als 30 Gew.-%, insbesondere weniger als 20 Gew.-% Ester der Formel Ib.

Im Hinblick auf die erfindungsgemäßen haftenden Zusammensetzungen sind solche Ester der Polyisobutenbernsteinsäure bevorzugt, deren Polyisobutenrest des Esters ein zahlenmittleres Molekulargewicht im Bereich von 500 bis 5000 Dalton, insbesondere im Bereich von 800 bis 3600 aufweist.

In einer speziellen Ausführungsform der Erfindung weisen Polyisobutenreste der Polyisobutenbernsteinsäureester eine enge Molekulargewichtsverteilung auf. Die Polydispersität beträgt dann vorzugsweise höchstens 1,4, besonders bevorzugt höchstens 1,3, insbesondere höchstens 1,2. Unter Polydispersität versteht man den Quotienten aus gewichtsmittlerem Molekulargewicht M_{w} und zahlenmittlerem Molekulargewicht Mₙ (PDI = M_{w}/Mₙ).

Im Hinblick auf die erfindungsgemäßen haftenden Zusammensetzungen sind solche Ester der Polyisobutenbernsteinsäure bevorzugt, die mit einem unter Poly-C₂-C₄-alkylenglykolen Poly-C₂-C₄-alkylenglykolmono-C₁-C₂₂-alkylether ausgewählten Alkohol oder einem Gemisch dieser Alkohole verestert sind, wobei der beziehungsweise die Alkohole ein zahlenmittleres Molekulargewicht im Bereich von 500 bis 15000 Dalton, insbesondere im Bereich von 800 bis 10000 Dalton und speziell im Bereich von 1200 bis 5000 Dalton aufweist beziehungsweise aufweisen.

Weiterhin hat es sich als vorteilhaft erwiesen, wenn der Alkohol, welcher mit der Polyisobutenbernsteinsäure verestert ist, unverzweigt ist, das heißt unter linearen Poly-C₂-C₄-alkylenglykolen und linearen Poly-C₂-C₄-alkylenglykolmono-C₁-C₂₀-alkylethern ausgewählt ist. Unverzweigte, das heißt lineare Poly-C₂-C₄-alkylenglykole und lineare Poly-C₂-C₄-alkylenglykolmono-C₁-C₂₀-alkylether können durch die folgende Formel (III) beschrieben werden:

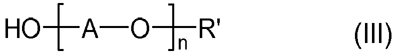

Hierin steht A für C₂-C₄-Alkandiyl wie zuvor definiert, das gleich oder verschieden sein kann und das vorzugsweise ausgewählt ist unter 1,2-Ethandiyl und 1,2-Propandiyl. R' steht für Wasserstoff oder C₁-C₂₂-Alkyl, insbesondere für Wasserstoff oder C₁-C₁₀-Alkyl und speziell für Wasserstoff oder C₁-C₄-Alkyl, zum Beispiel für Methyl. Die Variable n gibt die mittlere Anzahl an Wiederholungseinheiten [A-O] an (Zahlenmittel) und liegt typischerweise im Bereich von 10 bis 350, insbesondere im Bereich von 15 bis 200.

Dementsprechend steht der Rest Pag in den Formeln la und Ib vorzugsweise für einen Rest der Formel 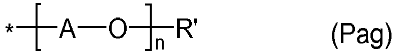 worin A, R und n die zuvor angegebenen Bedeutungen aufweisen und * die Verküpfung zum Sauerstoffatom des Polyisbutenbernsteinsäurerestes bedeutet.

In Formel III beziehungsweise in Formel Pag können die Wiederholungseinheiten der Formel -A-O-gleich oder verschieden sein. Sofern die Formeln III beziehungsweise in Formeln Pag verschiedene Wiederholungseinheiten der Formel -A-O- aufweisen, können diese statistisch oder in mehreren, zum Beispiel 2, 3 oder 4 Blöcken angeordnet sein. In einer bestimmten Ausführungsform der Erfindung weisen die Formeln III beziehungsweise in Formeln Pag unterschiedliche Wiederholungseinheiten der Formel -A-O- auf, die statistisch angeordnet sind. Weiterhin hat es sich als vorteilhaft erwiesen, wenn der Alkohol, welcher mit der Polyisobutenbernsteinsäure verestert ist, zu wenigstens 50 mol-%, und insbesondere zu wenigstens 70 mol-%, bezogen auf die Gesamtzahl an Alkylenoxid-Wiederholungseinheiten im Alkohol, aus Wiederholungseinheiten der Formel [CH₂CH₂O] aufgebaut ist. Dementsprechend beträgt in den Formeln III und Pag der Anteil an Wiederholungseinheiten der Formel [CH₂CH₂O] wenigstens 50 mol-%, und insbesondere wenigstens 70 mol-%, bezogen auf die Gesamtzahl an Wiederholungseinheiten A-O.

In einer speziellen Ausführungsform der Erfindung sind alle oder nahezu alle Wiederholungseinheiten A-O des Poly-C₂-C₄-alkylenglykole beziehungsweise des Poly-C₂-C₄-alkylenglykolmono-C₁-C₂₀-alkylethers, beziehungsweise alle oder nahezu alle Wiederholungseinheiten A-O in den Formeln III und Pag, Wiederholungseinheiten der Formel [CH₂CH₂O].

In einer weiteren bevorzugten Ausführungsform der Erfindung umfasst der Alkohol, welcher mit der Polyisobutenbernsteinsäure verestert ist, insbesondere der Alkohol der Formel III beziehungsweise der Rest Pag
- 50 mol-% bis 99 mol-%, und insbesondere 70 mol-% bis 98 mol-%, bezogen auf die Gesamtzahl an Alkylenoxid-Wiederholungseinheiten im Alkohol, Wiederholungseinheiten der Formel [CH₂CH₂O], sowie
- 1 mol-% bis 50 mol-%, und insbesondere 2 mol-% bis 30 mol-%, bezogen auf die Gesamtzahl an Alkylenoxid-Wiederholungseinheiten im Alkohol, Wiederholungseinheiten der Formel [A'-O], worin A' für C₃-C₄-Alkandiyl steht, und insbesondere Wiederholungseinheiten der Formel [CH₂CH(CH₃)O].

In einer speziellen Ausgestaltung dieser bevorzugten Ausführungsform sind die voneinander verschiedenen Wiederholungseinheiten [CH₂CH₂O] und [A'-O] nicht blockartig sondern statistisch verteilt oder alternierend angeordnet.

Ferner hat es sich als vorteilhaft erwiesen, wenn der Alkoholbestandteil und die dem Ester zugrunde liegende Polyisobutenbernsteinsäure so ausgewählt ist, dass der Ester im Mittel ein Gewichtsverhältnis von Polyisobutenrest zu Alkoholrest im Bereich von 10:1 bis 1:30, vorzugsweise im Bereich von 1,5:1 bis 1:20 und insbesondere im Bereich von 1:1 bis 1:10 aufweist.

Der Ester der Polyisobutenbernsteinsäure ist in einer Menge von 0,1 bis 80 Gew.-%, insbesondere von 2 bis 70 Gew.-%, besonders bevorzugt von 10 bis 60 Gew.-%, ganz besonders bevorzugt 20 bis 50 Gew.-% enthalten. Die Ester der Polyisobutenbernsteinsäure dienen erfindungsgemäß als Haftvermittler.

Als Haftvermittler können alternativ zu den Polyisobutenbernsteinsäureestern weiterhin anorganische Verdickungsmittel (Polykieselsäuren, Tonmineralien wie Montmorillonite, Zeolithe, Kieselsäuren) eingesetzt werden. Besonders geeignet sind hierbei Schichtsilikate. Hierzu zählen beispielsweise die unter dem Handelsnamen *Laponite*® erhältlichen Magnesium- oder Natrium-Magnesium- Schichtsilikate der Firma *Solvay Alkali,* insbesondere das *Laponite*® *RD* oder auch *Laponite*® *RDS,* sowie die Magnesiumsilikate der Firma *Süd-Chemie,* vor allem das *Optigel*® *SH.*

Die anorganischen Verdickungsmittel sind vorzugsweise in einer Menge von 0,1 bis 80 Gew.-%, vorzugsweise 10 bis 60 Gew.-%, besonders bevorzugt 20 bis 50 Gew.-%, jeweils bezogen auf die gesamte haftende Zusammensetzung, enthalten.

In einer Ausführungsform können weiterhin Ester der Polyisobutenbernsteinsäure gemeinsam mit anorganischen Verdickungsmitteln eingesetzt werden, insgesamt in einer Menge von bis zu 80 Gew.-%, vorzugsweise 10 bis 60 Gew.-%, besonders bevorzugt 20 bis 50 Gew.-%, jeweils bezogen auf die gesamte haftende Zusammensetzung.

### Parfüm

Die erfindungsgemäße Zusammensetzung enthält einen oder mehrere Duftstoffe, vorzugsweise in einer Menge von 0,01 bis 15 Gew.-%, insbesondere 0,05 bis 10 Gew.-%, besonders bevorzugt 0,1 bis 8 Gew.-%, jeweils bezogen auf die gesamte haftende Zusammensetzung. Als eine Parfümkomponente kann dabei d-Limonen enthalten sein. In einer besonders bevorzugten Ausführungsform enthält die Zusammensetzung dabei ein Parfüm aus ätherischen Ölen (auch als essentielle Öle bezeichnet). Als solche sind beispielsweise Pine-, Citrus-, Jasmin-, Patchouly-, Rosen- oder Ylang-Ylang-Öl im Sinne dieser Erfindung einsetzbar. Ebenfalls geeignet sind Muskateller-Salbeiöl, Kamillenöl, Lavendelöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Haftfeste Riechstoffe, die im Rahmen der vorliegenden Erfindung vorteilhafterweise in den Parfümölen einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lemongrasöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Sternanisöl, Thujaöl, Thymianöl, Verbenaöl, Vetiveröl, Wacholderbeeröl, Wermutöl, Wintergrünöl, Ylang -Ylang-Öl, Ysop-Öl, Zimtöl, Zimtblätteröl sowie Zypressenöl.

Aber auch die höhersiedenden beziehungsweise festen Riechstoffe natürlichen oder synthetischen Ursprungs können im Rahmen der vorliegenden Erfindung vorteilhafterweise als haftfeste Riechstoffe beziehungsweise Riechstoffgemische in den Parfümölen eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p- Kresolmethyl-ether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester.

Zu den leichter flüchtigen Riechstoffen, die im Rahmen der vorliegenden Erfindung in den Parfümölen vorteilhaft einsetzbar sind, zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Citral, Citronellal.

### Weitere Inhaltsstoffe

Neben den bisher genannten Komponenten kann die erfindungsgemäße Zusammensetzung weitere übliche Inhaltsstoffe von WC-Reinigungsmitteln enthalten, vorzugsweise ausgewählt aus der Gruppe umfassend Säuren, Basen, Salze, Verdickungsmittel, antimikrobielle Wirkstoffe, Konservierungsstoffe, Komplexbildner, Polymere, Farbstoffe, Parfümbooster, Füllstoffe, Builder, Bleichmittel, Bitterstoffe, Korrosionsinhibitoren, Enzyme, Mikroorganismen, Wirkstoffe zur Biofilmentfernung, Wirkstoffe zur Inhibierung der Kalkablagerung, Wirkstoffe zur Verminderung der Schmutzhaftung sowie Gemische derselben. Insgesamt sollten nicht mehr als 60 Gew.-% weitere Inhaltsstoffe enthalten sein, vorzugsweise 0,01 bis 40 Gew.-%, insbesondere 0,2 bis 35 Gew.-%.

### Säuren

Erfindungsgemäße Zusammensetzungen können zur Verstärkung der Reinigungsleistung gegenüber Kalk und Urinstein eine oder mehrere Säuren und/oder deren Salze enthalten. Bevorzugt werden die Säuren aus nachwachsenden Rohstoffen hergestellt. Als Säuren eignen sich daher insbesondere organische Säuren wie Essigsäure, Citronensäure, Glycolsäure, Milchsäure, Bernsteinsäure, Adipinsäure, Äpfelsäure, Weinsäure und Gluconsäure sowie Gemische derselben. Daneben kann aber auch Amidosulfonsäure eingesetzt werden. Besonders bevorzugt sind die Säuren und/oder ihre Salze ausgewählt aus der Gruppe umfassend Citronensäure, Milchsäure, Amidosulfonsäure, ihre Salze sowie Gemische derselben. Sie werden vorzugsweise in Mengen von 0,01 bis 10 Gew.-% eingesetzt, besonders bevorzugt 0,2 bis 5 Gew.-%.

Daneben kann die Zusammensetzung in einer Ausführungsform anorganische Salze enthalten, vorzugsweise Alkali- oder Erdalkalimetallsalze, insbesondere Carbonate, Sulfate, Halogenide oder Phosphate sowie Gemische derselben. Besonders bevorzugt werden Natriumsulfat und/oder Natriumcarbonat eingesetzt. Natriumsulfat kann dabei in einer Menge von bis zu 60 Gew.-% enthalten sein, vorzugsweise 0,01 bis 60 Gew.-%, besonders bevorzugt 20 bis 60 Gew.-%, insbesondere 35 bis 55 Gew.-%, jeweils bezogen auf die gesamte haftende Zusammensetzung. Natriumcarbonat und weitere Salze können in einer Menge von bis zu 30 Gew.-%, vorzugsweise bis zu 10 Gew.-%, besonders bevorzugt bis zu 5 Gew.-% , jeweils bezogen auf die gesamte haftende Zusammensetzung, enthalten sein.

### Basen

In erfindungsgemäßen Zusammensetzungen können weiterhin Alkalien enthalten sein. Als Basen werden in erfindungsgemäßen Mitteln vorzugsweise solche aus der Gruppe der Alkali- und Erdalkalimetallhydroxide und -carbonate, insbesondere Natriumcarbonat oder Natriumhydroxid, eingesetzt. Daneben können aber auch Ammoniak und/oder Alkanolamine mit bis zu 9 C-Atomen im Molekül verwendet werden, vorzugsweise die Ethanolamine, insbesondere Monoethanolamin.

### Antimikrobielle Wirkstoffe

Eine besondere Form der Reinigung stellen die Desinfektion und die Sanitation dar. In einer entsprechenden besonderen Ausführungsform der Erfindung enthält das Gel oder die Paste daher einen oder mehrere antimikrobielle Wirkstoffe, vorzugsweise in einer Menge von bis zu 40 Gew.-%, bevorzugt 0,01 bis 25 Gew.-%, insbesondere 0,1 bis 5 Gew.-%, jeweils bezogen auf die gesamte haftende Zusammensetzung.

Die Begriffe Desinfektion, Sanitation, antimikrobielle Wirkung und antimikrobieller Wirkstoff haben im Rahmen der erfindungsgemäßen Lehre die fachübliche Bedeutung. Während Desinfektion im engeren Sinne der medizinischen Praxis die Abtötung von - theoretisch allen - Infektionskeimen bedeutet, ist unter Sanitation die möglichst weitgehende Eliminierung aller - auch der für den Menschen normalerweise unschädlichen saprophytischen - Keime zu verstehen. Hierbei ist das Ausmaß der Desinfektion beziehungsweise Sanitation von der antimikrobiellen Wirkung des angewendeten Mittels abhängig, die mit abnehmendem Gehalt an antimikrobiellem Wirkstoff beziehungsweise zunehmender Verdünnung des Mittels zur Anwendung abnimmt.

Erfindungsgemäß geeignet sind beispielsweise antimikrobielle Wirkstoffe aus den Gruppen der Alkohole, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazole und deren Derivate wie Isothiazoline und Isothiazolinone, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propynyl-butyl-carbamat, lod, lodophore, Aktivchlor abspaltenden Verbindungen und Peroxide. Bevorzugte antimikrobielle Wirkstoffe werden vorzugsweise ausgewählt aus der Gruppe umfassend 1,3-Butandiol, Phenoxyethanol, 1,2-Propylenglykol, Glycerin, Undecylensäure, Citronensäure, Milchsäure, Benzoesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraazatetradecandiimidamid, antimikrobielle quaternäre oberflächenaktive Verbindungen, Guanidine, Trichloroisocyanursäure und Natrium-Dichlorisocyanurat (DCI, 1,3-Dichlor-5H-1,3,5-triazin-2,4,6-trion Natriumsalz). Bevorzugte antimikrobiell wirkende oberflächenaktive quaternäre Verbindungen enthalten eine Ammonium-, Sulfonium-, Phosphonium-, Jodonium- oder Arsoniumgruppe. Weiterhin können auch antimikrobiell wirksame ätherische Öle eingesetzt werden, die gleichzeitig für eine Beduftung des Reinigungsmittels sorgen. Besonders bevorzugte antimikrobielle Wirkstoffe sind jedoch ausgewählt aus der Gruppe umfassend Salicylsäure, quaternäre Tenside, insbesondere Benzalkoniumchlorid, PeroxoVerbindungen, insbesondere Natriumpercarbonat oder Phthalimidoperoxyhexanoic acid, Alkalimetallhypochlorit, Trichloroisocyanursäure, Natriumdichlorisocyanurat sowie Gemische derselben.

### Konservierungsstoffe

Konservierungsstoffe können gleichfalls in erfindungsgemäßen Zusammensetzungen enthalten sein. Als solche können im Wesentlichen die bei den antimikrobiellen Wirkstoffen genannten Stoffe eingesetzt werden.

### Komplexbildner

Komplexbildner (*INCI* Chelating Agents), auch Sequestriermittel genannt, sind Inhaltsstoffe, die Metallionen zu komplexieren und inaktivieren vermögen, um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert andererseits die oxidative Zersetzung der fertigen Mittel. Zudem unterstützen die Komplexbildner die Reinigungswirkung.

Geeignet sind beispielsweise die folgenden gemäß *INCI* bezeichneten Komplexbildner: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphosphate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

### Polymere

Die erfindungsgemäße haftende Zusammensetzung kann weiterhin Polymere enthalten. Diese können beispielsweise zur Verringerung der Kalkbildung sowie der Wiederanschmutzungsneigung dienen. Bevorzugte Polymere sind dabei Acrylcopolymere, wie sie etwa von der Firma Rhodia unter dem Handelsnamen Mirapol® kommerziell erhältlich sind.

### Farbstoffe

Als weitere Inhaltsstoffe kann die erfindungsgemäße Zusammensetzung einen oder mehrere Farbstoffe (*INCI* Colorants) enthalten. Als Farbstoffe können dabei sowohl wasserlösliche als auch öllösliche Farbstoffe verwendet werden, wobei einerseits die Kompatibilität mit weiteren Inhaltsstoffen, beispielsweise Bleichmitteln, zu beachten ist und andererseits der eingesetzte Farbstoff gegenüber der WC-Keramik auch bei längerem Einwirken nicht substantiv wirken sollte. Zudem sollte es sich bei den Farbstoffen nicht um Pigmentdispersionen in mehrwertigen Alkoholen oder Gemischen aus mehrwertigen Alkoholen und Wasser handeln. Die Farbstoffe sind vorzugsweise in einer Menge von 0,0001 bis 5 Gew.-%, insbesondere 0,0005 bis 0,25 Gew.-%, besonders bevorzugt 0,0008 bis 0,08 Gew.-%,jeweils bezogen auf die gesamte haftende Zusammensetzung, enthalten.

### Builder

In den erfindungsgemäßen Zusammensetzungen können gegebenenfallswasserlösliche und/oder wasserunlösliche Builder eingesetzt werden. Dabei sind wasserlösliche Builder bevorzugt, da sie in der Regel weniger dazu tendieren, auf harten Oberflächen unlösliche Rückstände zu hinterlassen. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildern zählen die Zeolithe, die ebenfalls verwendet werden können, ebenso wie Mischungen der vorgenannten Buildersubstanzen.

### Bleichmittel

Erfindungsgemäß können Bleichmittel dem Reinigungsmittel zugesetzt werden. Geeignete Bleichmittel umfassen Peroxide, Persäuren und/oder Perborate, besonders bevorzugt ist Natriumpercarbonat oder Phthalimidoperoxyhexanoic acid. Chlorhaltige Bleichmittel wie Trichlorisocyanursäure oder Natriumdichlorisocyanurat sind dagegen bei sauer formulierten Reinigungsmitteln aufgrund der Freisetzung giftiger Chlorgas-Dämpfe weniger geeignet, können jedoch in alkalisch eingestellten Reinigungsmitteln eingesetzt werden. Unter Umständen kann neben dem Bleichmittel auch ein Bleichaktivator vonnöten sein.

### Korrosionsinhibitoren

Geeignete Korrosionsinhibitoren (*INCI* Corrosion Inhibitors) sind beispielsweise folgende gemäß *INCI* benannte Substanzen: Cyclohexylamine, Diammonium Phosphate, Dilithium Oxalate, Dimethylamino Methylpropanol, Dipotassium Oxalate, Dipotassium Phosphate, Disodium Phosphate, Disodium Pyrophosphate, Disodium Tetrapropenyl Succinate, Hexoxyethyl Diethylammonium, Phosphate, Nitromethane, Potassium Silicate, Sodium Aluminate, Sodium Hexametaphosphate, Sodium Metasilicate, Sodium Molybdate, Sodium Nitrite, Sodium Oxalate, Sodium Silicate, Stearamidopropyl Dimethicone, Tetrapotassium Pyrophosphate, Tetrasodium Pyrophosphate, Triisopropanolamine.

### Enzyme

Die Zusammensetzung kann auch Enzyme enthalten, vorzugsweise Proteasen, Lipasen, Amylasen, Hydrolasen und/oder Cellulasen. Sie können dem erfindungsgemäßen Mittel in jeder nach dem Stand der Technik etablierten Form zugesetzt werden. Hierzu gehören Lösungen der Enzyme, vorteilhafterweise möglichst konzentriert, wasserarm und/oder mit Stabilisatoren versetzt. Alternativ können die Enzyme verkapselt werden, beispielsweise durch Sprühtrocknung oder Extrusion der Enzymlösung zusammen mit einem, vorzugsweise natürlichen, Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil. Weiterhin können in enzymhaltigen Mitteln Enzymstabilisatoren vorhanden sein, um ein in einem erfindungsgemäßen Mittel enthaltenes Enzym vor Schädigungen wie beispielsweise Inaktivierung, Denaturierung oder Zerfall etwa durch physikalische Einflüsse, Oxidation oder proteolytische Spaltung zu schützen. Als Enzymstabilisatoren sind, jeweils in Abhängigkeit vom verwendeten Enzym, insbesondere geeignet: Benzamidin-Hydrochlorid, Borax, Borsäuren, Boronsäuren oder deren Salze oder Ester, vor allem Derivate mit aromatischen Gruppen, etwa substituierte Phenylboronsäuren beziehungsweise deren Salze oder Ester; Peptidaldehyde (Oligopeptide mit reduziertem C-Terminus), Aminoalkohole wie Mono-, Di-, Triethanol- und -Propanolamin und deren Mischungen, aliphatische Carbonsäuren bis zu C₁₂, wie Bernsteinsäure, andere Dicarbonsäuren oder Salze der genannten Säuren; endgruppenverschlossene Fettsäureamidalkoxylate; niedere aliphatische Alkohole und vor allem Polyole, beispielsweise Glycerin, Ethylenglykol, Propylenglykol oder Sorbit; sowie Reduktionsmittel und Antioxidantien wie Natriumsulfit und reduzierende Zucker. Weitere geeignete Stabilisatoren sind aus dem Stand der Technik bekannt. Bevorzugt werden Kombinationen von Stabilisatoren verwendet, beispielsweise die Kombination aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen.

Die Zusammensetzung wird vorteilhafterweise mit einem Applikator aufgetragen. Es kann aus ästhetischen Gründen, aber auch, um miteinander unverträgliche Wirkstoffe gemeinsam zur Anwendung bringen zu können, wünschenswert sein, gleichzeitig zwei oder sogar mehr Portionen des erfindungsgemäßen haftenden Gels oder der Paste nebeneinander auf die WC-Keramik aufzutragen. Hierzu eignet sich in besonderer Weise ein Zweikammer-Applikator, wie er auch in der Patentanmeldung DE 102010028352.5 beschrieben wird. Dieser Applikator kann in seinen beiden Kammern Gele oder Pasten gleicher oder unterschiedlicher Zusammensetzung bevorraten.

Bei der Anwendung wird mit dem Applikator üblicherweise eine Menge von 4 bis 8 g der Zusammensetzung auf die WC-Keramik aufgetragen. Diese Auftragsmenge ist vorteilhafterweise nach 100 bis 200 Spülvorgängen vollständig abgelöst.

Das erfindungsgemäße Mittel wird hergestellt, indem die einzelnen Komponenten gemischt und anschließend abgefüllt werden. Als Mischer kommen dabei alle Maschinen in Frage, die viskose Massen verarbeiten können, beispielsweise SpeedMixer, Planetenmischer oder Statikmischer. Um Verluste an flüchtigen Inhaltsstoffen, insbesondere beim Parfüm, so gering wie möglich zu halten, erfolgt die Verarbeitung bei möglichst niedriger Temperatur, maximal aber bei 85°C. Bei dieser Temperatur wird der Polyisobutenbernsteinsäureester aufgeschmolzen und unter Rühren im Mischer nacheinander Tenside, Parfümöl und weitere Komponenten zugegeben. Im warmen Zustand ist die Formulierung fließfähig und kann abgefüllt werden.
Formulierungen, die frei von Polyisobutenbernsteinsäureester sind, können hergestellt werden, indem das Wasser vorgelegt und feste Bestandteile, etwa Laponit, unter Rühren mittels einer Dispergierscheibe (Meiserscheibe)zugegeben werden. Anschließend werden Tenside und Duftstoffe und abschließend ein Gemisch aus Farbstoff, Wasser und Bitterstoffen sowie gegebenenfalls weiteren flüssigen Bestandteilen zugegeben. Alternativ können sämtliche flüssige Komponenten vorgelegt und die festen Inhaltsstoffe über eine Hochschereinrichtung zugegeben werden, wobei neben einer Dispergierscheibe auch Rotor-Stator-Systeme (batch oder inline) in Frage kommen.

### Ausführungsbeispiele

Es wurden zwei erfindungsgemäße gelförmige Formulierungen E1 und E2 hergestellt, die jeweils als mehrphasiges Mittel, bestehend aus einer Parfümphase und einer Reinigungsphase, in einen Zweikammerapplikator abgefüllt wurden. Die Zusammensetzungen sind der nachfolgenden Tabelle zu entnehmen. Die Mengenangaben sind dabei in Gew.-% Aktivsubstanz.

| | E1 | | E2 | |
|---|---|---|---|---|
| | Duft | Reinigung | Duft | Reinigung |
| Ester der Polyisobutenbernsteinsäure | 40,00 | 40,00 | -- | -- |
| Hostapur® SAS 60 | 30,10 | 30,10 | 40,50 | 40,50 |
| Laponite® TM | -- | -- | 25,00 | 25,00 |
| Parfümöl | 4,90 | 4,90 | 4,90 | 4,90 |
| Bitterstoff (Bitrex®) | 0,0010 | 0,0010 | 0,0010 | 0,0010 |
| Trinatriumcitrat | -- | 0,10 | -- | 0,10 |
| Farbstoff gelb | 0,0150 | -- | 0,0150 | -- |
| Farbstoff blau | -- | 0,0130 | -- | 0,0130 |
| Wasser (vollentsalzt) | ad 100 | ad 100 | ad 100 | ad 100 |

Beim Hostapur® SAS 60 handelt es sich um ein sekundäres C₁₃₋₁₇-Alkansulfonat ex Clariant.

Die erfindungsgemäßen Formulierungen haften gut auf der WC-Keramik und weisen beide eine Reichweite von ca. 120 Spülungen auf.

## Patentansprüche

1. Haftende gelförmige oder pastöse Zusammensetzung zur Reinigung und/oder Beduftung eines WCs, welche innen auf die WC-Keramik aufgetragen und erst nach mehreren Spülvorgängen abgespült wird, enthaltend
- mindestens ein Tensid aus der Gruppe bestehend aus Fettalkoholsulfaten, Fettalkoholethersulfaten, Alkylsulfonaten, Sulfo-Estoliden, Methylestersulfonaten, Acylaminoalkansulfonaten (Fettsäuretauriden), Sulfosuccinaten sowie Gemischen derselben
- Parfüm und
- Wasser,
**dadurch gekennzeichnet, dass** sie
- 0,1 bis 80 Gew.-% eines Esters der Polyisobutenbernsteinsäure enthält und/oder
- frei von weiteren Tensiden sowie von aliphatischen Verbindungen mit mehr als einer OH-Gruppe ist.

2. Haftende Zusammensetzung gemäß Anspruch 1, **dadurch gekennzeichnet, dass** der Ester der Polyisobutenbernsteinsäure vorzugsweise in einer Menge von 10 bis 60 Gew.-%, besonders bevorzugt 20 bis 50 Gew.-%, jeweils bezogen auf die gesamte haftende Zusammensetzung, enthalten ist.

3. Haftende Zusammensetzung gemäß einem der Ansprüche 1 oder 2, **dadurch gekennzeichnet, dass** das Tensid ausgewählt ist aus der Gruppe bestehend aus Fettalkoholsulfaten, Fettalkoholethersulfaten, sek. Alkansulfonaten, Sulfo-Estoliden, Methylestersulfonaten, Acylaminoalkansulfonaten (Fettsäuretauriden), Fettsäuresarcosinaten, Sulfosuccinaten sowie Gemischen derselben.

4. Haftende Zusammensetzung nach einem der vorangehenden Ansprüche, **dadurch gekennzeichnet, dass** sie das mindestens eine Tensid in einer Menge von 10 bis 80 Gew.-%, vorzugsweise 20 bis 60 Gew.-%, besonders bevorzugt 25 bis 55 Gew.-%, jeweils bezogen auf die gesamte haftende Zusammensetzung enthält.

5. Haftende Zusammensetzung nach einem der vorangehenden Ansprüche, **dadurch gekennzeichnet, dass** sie weiterhin ein anorganisches Verdickungsmittel, vorzugsweise ein Schichtsilikat, in einer Menge von 0 bis 80 Gew.-%, vorzugsweise 10 bis 60 Gew.-%, besonsers bevorzugt 20 bis 50 Gew.-%, jeweils bezogen auf die gesamte haftende Zusammensetzung enthält.

6. Haftende Zusammensetzung gemäß einem der vorangehenden Ansprüche, **dadurch gekennzeichnet, dass** sie weitere übliche Inhaltsstoffe von WC-Reinigungsmitteln enthält, vorzugsweise ausgewählt aus der Gruppe umfassend Säuren, Basen, Salze, Verdickungsmittel, antimikrobielle Wirkstoffe, Konservierungsstoffe, Komplexbildner, Farbstoffe, Parfümbooster, Füllstoffe, Builder, Bleichmittel, Bitterstoffe, Korrosionsinhibitoren, Enzyme, Mikroorganismen, Wirkstoffe zur Biofilmentfernung, Wirkstoffe zur Inhibierung der Kalkablagerung, Wirkstoffe zur Verminderung der Schmutzhaftung sowie Gemische derselben.

7. Haftende Zusammensetzung gemäß einem der vorangehenden Ansprüche, **dadurch gekennzeichnet, dass** eine Auftragsmenge von 4 bis 8 g nach 100 bis 200 Spülvorgängen vollständig abgelöst ist

8. Haftende Zusammensetzung gemäß einem der vorangehenden Ansprüche, **dadurch gekennzeichnet, dass** sie mittels eines Applikators auf die WC-Keramik aufgetragen wird und dort haftet.

9. Haftende Zusammensetzung gemäß Anspruch 8, **dadurch gekennzeichnet, dass** mit dem Applikator gleichzeitig zwei Portionen nebeneinander aufgetragen werden.

10. Haftende Zusammensetzung gemäß Anspruch 9, **dadurch gekennzeichnet, dass** der Applikator zwei Kammern aufweist, in denen Gele oder Pasten gleicher oder unterschiedlicher Zusammensetzung bevorratet werden.

## Claims

1. An adhesive gel-like or pasty composition for cleansing and/or fragrancing a toilet, which is applied to the interior of the toilet ceramic and only washed off after a plurality of flushes, containing
- at least one surfactant from the group consisting of fatty alcohol sulfates, fatty alcohol ether sulfates, alkyl sulfonates, sulfo-estolides, methyl ester sulfonates, acylamino alkane sulfonates (fatty acid taurides), sulfosuccinates and mixtures thereof,
- perfume and water,
**characterized in that** it
- contains 0.1 to 80 wt.% of an ester of polyisobutene succinic acid and/or
- is free from further surfactants and from aliphatic compounds which have more than one OH group.

2. The adhesive composition according to claim 1, **characterized in that** the ester of polyisobutene succinic acid is preferably contained in an amount of from 10 to 60 wt.%, particularly preferably 20 to 50 wt.%, in each case based on the entire adhesive composition.

3. The adhesive composition according to one of claims 1 or 2, **characterized in that** the surfactant is selected from the group consisting of fatty alcohol sulfates, fatty alcohol ether sulfates, secondary alkane sulfonates, sulfo-estolides, methyl ester sulfonates, acylamino alkane sulfonates (fatty acid taurides), fatty acid sarcosinates, sulfosuccinates and mixtures thereof.

4. The adhesive composition according to one of the preceding claims, **characterized in that** it contains the at least one surfactant in an amount of from 10 to 80 wt.%, preferably 20 to 60 wt.%, particularly preferably 25 to 55 wt.%, in each case based on the entire adhesive composition.

5. The adhesive composition according to one of the preceding claims, **characterized in that** it also contains an inorganic thickening agent, preferably a phyllosilicate, in an amount of from 0 to 80 wt.%, preferably 10 to 60 wt.%, particularly preferably 20 to 50 wt.%, in each case based on the entire adhesive composition.

6. The adhesive composition according to one of the preceding claims, **characterized in that** it contains further ingredients typical of toilet cleaning agents, preferably selected from the group comprising acids, bases, salts, thickening agents, antimicrobial active ingredients, preservatives, complexing agents, dyes, perfume boosters, fillers, builders, bleaching agents, bitterns, corrosion inhibitors, enzymes, microorganisms, active ingredients for removing biofilm, active ingredients for inhibiting limescale build-up, active ingredients for reducing dirt adhesion and mixtures thereof.

7. The adhesive composition according to one of the preceding claims, **characterized in that** an applied quantity of from 4 to 8 g is fully dissolved after 100 to 200 flushes.

8. The adhesive composition according to one of the preceding claims, **characterized in that** it is applied to the toilet ceramic by means of an applicator and adheres thereto.

9. The adhesive composition according to claim 8, **characterized in that** two portions are applied side by side simultaneously using the applicator.

10. The adhesive composition according to claim 9, **characterized in that** the applicator has two chambers, in which gels or pastes of the same composition or differing compositions are stored.

## Revendications

1. Composition gélatineuse ou pâteuse adhésive destinée à nettoyer et/ou parfumer des toilettes, laquelle composition est appliquée à l'intérieur de la cuvette des toilettes et rincée seulement après plusieurs processus de rinçage, la composition contenant
- au moins un tensioactif du groupe comportant les sulfates d'alcools gras, les sulfonates d'alkyle, les sulfo-estolides, les sulfonates d'ester méthylique, les sulfonates d'acylaminoalkane (taurides d'acides gras), les sulfosuccinates et leurs mélanges
- un parfum et
- de l'eau,
**caractérisée en ce que**
- elle contient de 0,1 à 80% en poids d'un ester d'acide polyisobutène-succinique et/ou
- elle est exempte d'autres tensioactifs et de composés aliphatiques comportant plus d'un groupe OH.

2. Composition adhésive selon la revendication 1, **caractérisée en ce que** l'ester de l'acide polyisobutène- succinique est de préférence contenu dans une quantité allant de 10 à 60% en poids, de manière particulièrement préférée de 20 à 50% en poids, à chaque fois sur la base de la composition adhésive totale.

3. Composition adhésive selon l'une des revendications 1 ou 2, **caractérisée en ce que** le tensioactif est choisi dans le groupe comportant les sulfates d'alcools gras, les éther-sulfates d'alcools gras, secondairement les sulfonates d'alcane, les sulfo-estolides, les ester-sulfonates de méthyle, les sulfonates d'acylaminoalcanes (les taurides d'acides gras), les sarcosinates d'acides gras, les sulfosuccinates et leurs mélanges.

4. Composition adhésive selon l'une des revendications précédentes, **caractérisé en ce qu'**elle comprend au moins un tensioactif dans une quantité allant de 10 à 80% en poids, de préférence de 20 à 60% en poids, de manière particulièrement préférée de 25 à 55% en poids, à chaque sur la base de la composition adhésive totale.

5. Composition adhésive selon l'une des revendications précédentes, **caractérisée en ce qu'**elle contient en outre un agent épaississant minéral, de préférence un phyllosilicate, dans une quantité de 0 à 80% en poids, de préférence de 10 à 60% en poids, de manière particulièrement préférée de 20 à 50% en poids, à chaque sur la base de la composition adhésive totale.

6. Composition adhésive selon l'une des revendications précédentes, **caractérisée en ce qu'**elle contient d'autres ingrédients typiques des agents de nettoyage de toilettes, choisis de préférence dans le groupe comportant des acides, des bases, des sels, des agents épaississants, des agents antimicrobiens, des conservateurs, des agents complexants, des colorants, des renforçateurs de parfum, des charges, des adjuvants, des agents de blanchiment, des agents d'amertume, des inhibiteurs de corrosion, des enzymes, des micro-organismes, des médicaments pour l'élimination de biofilm, des agents d'inhibition de la calcification, des agents de réduction d'adhérence de souillures et leurs mélanges.

7. Composition adhésive selon l'une des revendications précédentes, **caractérisée en ce qu'**une quantité d'application de 4 à 8 g est complètement détachée au bout de 100 à 200 processus de rinçage.

8. Composition adhésive selon l'une des revendications précédentes, **caractérisée en ce qu'**elle est appliquée au moyen d'un applicateur sur la céramique des toilettes où elle adhère.

9. Composition adhésive selon la revendication 8, **caractérisée en ce que** l'applicateur applique simultanément deux portions côte à côte.

10. Composition adhésive selon la revendication 9, **caractérisée en ce que** l'applicateur comprend deux chambres dans lesquelles sont stockés des gels ou des pâtes de compositions identiques ou différentes.
